# FASCICULE DE BREVET EUROPEEN

(11) **EP 2 062 610 B1**
(45) Date de publication et mention de la délivrance du brevet: **10.07.2013**
(21) Numéro de dépôt: 08169438.2
(22) Date de dépôt: 19.11.2008
(51) Int. Cl.: A61M 25/10

(54) **Dispositif de gonflage de ballonet**
Vorrichtung zum Aufblasen eines Luftsacks
Device for inflating a small balloon

(30) Priorité: 20.11.2007 FR 0759171
(43) Date de publication de la demande: 27.05.2009
(73) Titulaire: SEDAT, 69540 Irigny (Rhône) (FR)
(72) Inventeur: Caclin, Jérôme, 69200 Venissieux (FR)
(74) Mandataire: Blot, Philippe Robert Emile

(56) Documents cités:
- EP-A- 1 440 706
- US-A- 5 019 041
- US-A- 5 470 317

## Description

La présente invention concerne un dispositif de gonflage de ballonne selon la revendication 1, du type comportant une seringue comprenant un corps de seringue et un piston de seringue déplaçable à coulissement et à rotation dans ledit corps de seringue, le piston présentant un filetage extérieur sur au moins une partie de sa longueur, le corps de seringue comportant en outre un mécanisme escamotable de retenue du piston dans le corps de seringue, lequel mécanisme est commutable entre un état escamoté dans lequel le piston est libre de coulisser dans le corps de seringue et un état actif dans lequel le libre coulissement du piston est impossible et dans lequel le piston peut être vissé ou dévissé dans le corps de seringue, le dispositif comportant un manomètre de mesure de la pression en sortie du corps de seringue.

Ces dispositifs de gonflage sont utilisés afin d'injecter un fluide dans un ballonnet préalablement disposé à l'état contracté à l'intérieur d'une artère ou d'une veine d'un malade. De tels dispositifs sont décrits par exemple dans les documents US-A-5,147,300, FR-2 850 286, EP 1 440 706, US 5 470 317 ou US 5 019 041.

Ces dispositifs sont en particulier utilisés pour les angioplasties transluminales percutanées, afin notamment de dilater l'artère ou la veine dans laquelle le ballonnet est disposé. Ils peuvent être utilisés pour d'autres applications.

Les dispositifs de gonflage doivent être capables de fournir une pression élevée, de l'ordre de 30 bars, cette pression étant suivie sur le manomètre du dispositif prévu à cet effet. Sous l'action une telle pression, il convient que la demi-noix coopérant avec le filetage soit maintenue fermement en main par l'opérateur et que celui-ci visse le piston avec son autre main. Cette manipulation nécessite que l'opérateur puisse prendre en main le corps du dispositif de la manière la plus commode pour lui. Ainsi, certains opérateurs choisissent de tenir le dispositif de sorte que l'organe de libération du piston soit au dessous du pouce alors que d'autres choisissent que cet organe soit au dessous de l'index. Certaines positions rendent l'accès visuel au manomètre difficile.

L'invention a pour but de fournir un dispositif de gonflage de ballonnet, qui présente une meilleure ergonomie de manipulation par un accès visuel amélioré au manomètre.

A cet effet, l'invention a pour objet un dispositif de gonflage de ballonnet du type précité, caractérisé en ce que le manomètre est monté déplaçable angulairement par rapport au corps de seringue et autour de l'axe du corps de seringue.

Suivant d'autres caractéristiques de ce dispositif, prises isolément ou selon toutes les combinaisons techniquement possibles :
- le dispositif comporte une coiffe délimitant un conduit traversant dans lequel débouche une prise de pression, le manomètre étant connecté en sortie de la prise de pression, et la coiffe est montée déplaçable en rotation autour du corps de seringue ;
- le dispositif comporte un joint torique d'étanchéité disposé entre la coiffe et le corps de seringue ;
- la coiffe est engagée élastiquement autour du corps de seringue ;
- le corps de seringue comporte un tronc généralement cylindrique prolongé par un goulot de diamètre extérieur inférieur à celui du tronc, et la coiffe comporte une jupe engagée au moins partiellement autour du tronc ;
- le dispositif comporte des butées d'arrêt du manomètre par rapport au corps de seringue limitant le déplacement angulaire du manomètre à sensiblement 90°;
- le dispositif comporte un module de pression dans lequel le manomètre est intégré, lequel module de pression est monté déplaçable angulairement par rapport au corps de seringue, et autour du corps de seringue, et il comporte en outre une tubulure de sortie reliée au module de pression par un embout rotatif libre en rotation par rapport au module de pression ;
- le corps de seringue comportant en outre un mécanisme escamotable de retenue du piston dans le corps de seringue, lequel mécanisme est commutable entre un état escamoté dans lequel le piston est libre de coulisser dans le corps de seringue et un état actif dans lequel le libre coulissement du piston est impossible et dans laquelle ce piston peut être vissé ou dévissé dans le corps de seringue ;
- le dispositif comportant un manomètre de mesure de la pression en sortie du corps de seringue ;
- le manomètre est monté angulairement par rapport au corps de seringue et autour de l'axe du corps de seringue ;
- le mécanisme de retenue comporte, pour la demi-noix, au moins un élément déformable élastiquement, en appui sur la demi-noix correspondante et sur le corps de seringue ; et
- l'organe de commande comporte au moins un doigt d'appui sur la demi-noix correspondante, adapté pour s'appuyer, lors du déplacement de l'organe de commande, contre deux surfaces portées par la demi-noix et décalées l'une par rapport à l'autre suivant une direction radiale au corps de seringue, la demi-noix étant dans sa position écartée du filetage lorsque le doigt est en appui contre la surface la plus proche radialement du piston de seringue et la demi-noix étant dans sa position en prise avec le filetage lorsque le doigt est en appui contre la surface la plus éloignée.

L'invention sera mieux comprise à la lecture de la description qui va suivre, donnée uniquement à titre d'exemple et fait en se référant aux dessins sur lesquels :
- la figure 1 est une vue en perspective partiellement éclatée du dispositif ;
- les figures 2 et 3 sont des vues en perspective d'un dispositif suivant l'invention, dans des configurations permettant respectivement le coulissement du piston de seringue et le vissage/dévissage de ce piston ;
- la figure 4 est une vue en perspective et en coupe longitudinale selon un plan médian du dispositif de la figure 2 ;
- La figure 5 est une vue plane en coupe selon le même plan que la coupe de la figure 4, du dispositif de la figure 2 ;
- La figure 6 est une vue en perspective à plus grande échelle d'une demi-noix contenue dans le dispositif ;
- La figure 7 est une vue en perspective à plus grande échelle d'un organe de commande de la demi-noix ;
- Les figures 8 et 9 sont des vues à plus grande échelle du détail de la coopération de la demi-noix et de l'organe de commande dans les configurations permettant respectivement le coulissement du piston et le vissage/dévissage de ce piston ;
- La figure 10 est une vue en perspective du dispositif de la figure 4, l'organe de commande du déplacement de la demi-noix n'étant pas représenté ; et
- Les figures 11 et 12 sont des vues en perspective du dispositif de la figure 1, avec son manomètre dans deux positions distinctes.

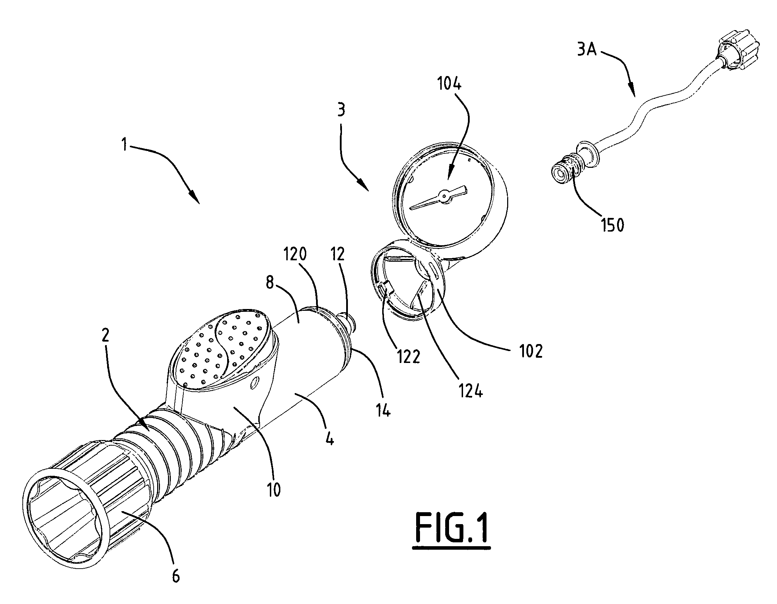

Le dispositif 1 de gonflage de ballonnet représenté sur les figures 1, 2, 3, 4, 5 comporte une seringue 2, un module de mesure de pression 3 et une tubulure 3A.

La seringue 2 s'étend le long d'un axe X-X et comprenant essentiellement un corps de seringue 4 et un piston 6. La seringue a avantageusement une contenance de 20 cm³.

Le corps de seringue 4, réalisé en matière plastique transparente, comporte un tronc 8 depuis lequel un boîtier latéral 10 venu de matière avec le tronc fait saillie. Le boîtier 10 est ouvert.

L'extrémité avant du corps de seringue présente un goulot 12 de plus petit diamètre prévu à l'extrémité d'un tronçon convergent 14 prolongeant le tronc. Cet ensemble de fixation comporte un organe déformable 14 de retenue du connecteur et des moyens d'étanchéité 16 formés par exemple d'un joint.

Le piston de seringue 6 est formé d'une tige 20 munie à son extrémité reçue à l'intérieur du tronc 8, au travers de l'extrémité arrière du corps de seringue 4, d'une tête 22 équipée d'un joint 23 qui coulisse de manière étanche à l'intérieur du corps de seringue 4. La tige comporte, à son autre extrémité, une poignée 24 destinée à l'actionnement manuel du piston 6. Cette poignée est pourvue d'un profil agrippant 26 pour faciliter sa prise en main.

La tige 20 est pourvue extérieurement d'un filetage 28 sur au moins une partie de sa longueur.

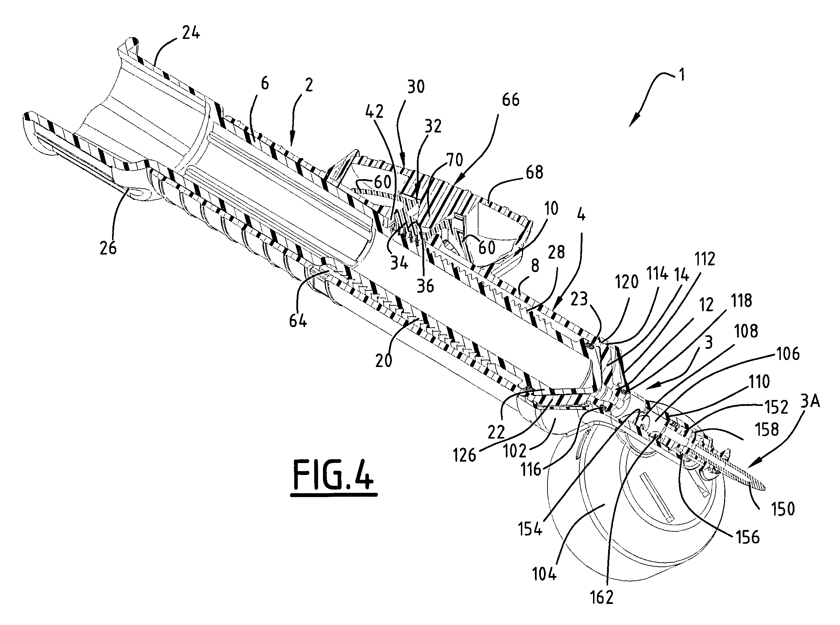

Le dispositif 1 comprend également un mécanisme 30 de retenue du piston 6, reçu pour l'essentiel à l'intérieur du boîtier latéral 10, comme représenté sur les figures 4, 5, 8 et 9 notamment.

L'ensemble de retenue 30 comporte une demi-noix 32 comprenant un corps creux 34, représenté plus en détail sur les figures 4, 5, 6, 8 et 9. Ce corps est essentiellement formé d'une paroi de fond 36 qui s'étend parallèlement au piston de seringue 6, deux parois transversales opposées 38 qui s'étendent sensiblement perpendiculairement à l'axe X-X de la seringue, et deux parois latérales opposées 40 qui relient les parois 38, une seule de ces parois 40 étant visible sur les figures 4, 5, 6, 8 et 9.

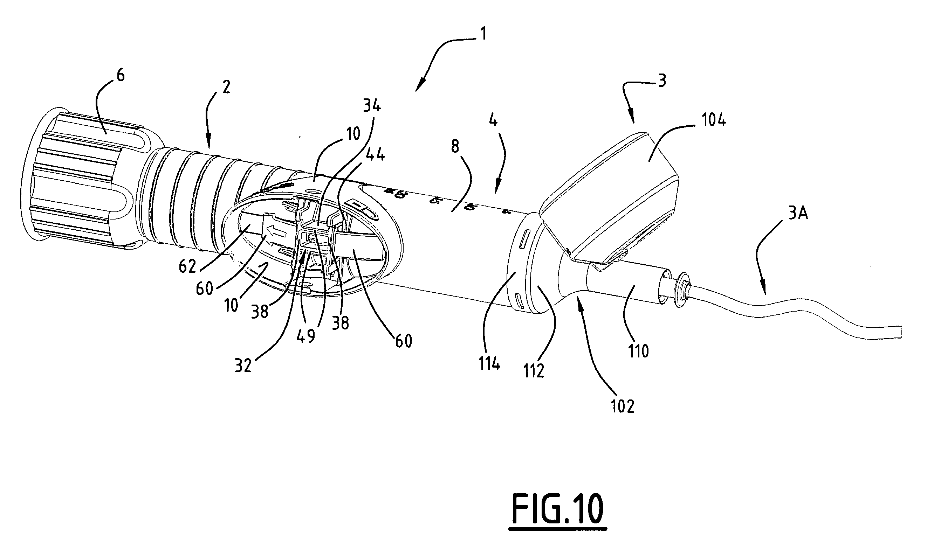

La demi-noix 32 est reçue à l'intérieur d'une ouverture oblongue traversante 42 ménagée dans le tronc 8 du corps de seringue 4, et y est maintenue par une console de logement 44 solidaire du corps de seringue 4, par exemple venue de matière avec le tronc 8 et le boîtier 10 comme illustré sur la figure 10. Cette console 44 est nervurée de façon, d'une part, à autoriser le déplacement de la demi-noix suivant une direction sensiblement perpendiculaire à l'axe X-X et contenue dans le plan de coupe de la figure 4, et, d'autre part, bloquer la demi-noix à la fois suivant les autres directions perpendiculaires à l'axe X-X et parallèlement à cet axe.

La paroi de fond 36 de la demi-noix 32 présente, du côté dirigé vers l'intérieur du corps de seringue 4, une surface taraudée 46 destinée à venir en prise avec le filetage 28 du piston 6.

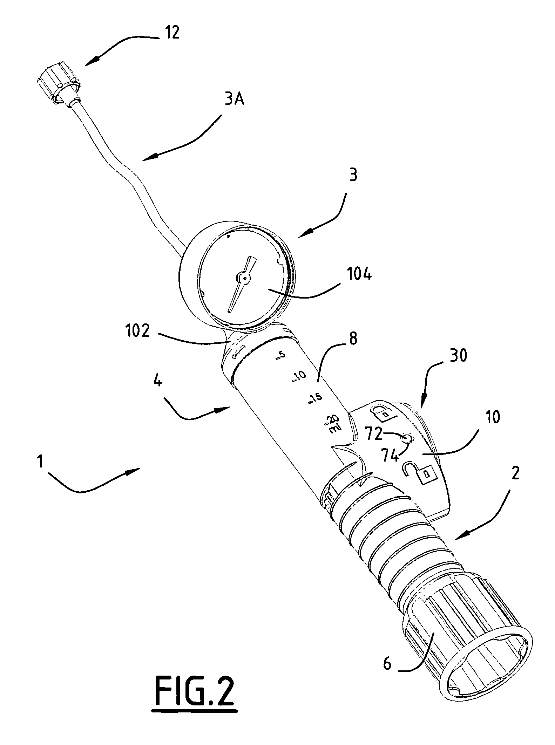

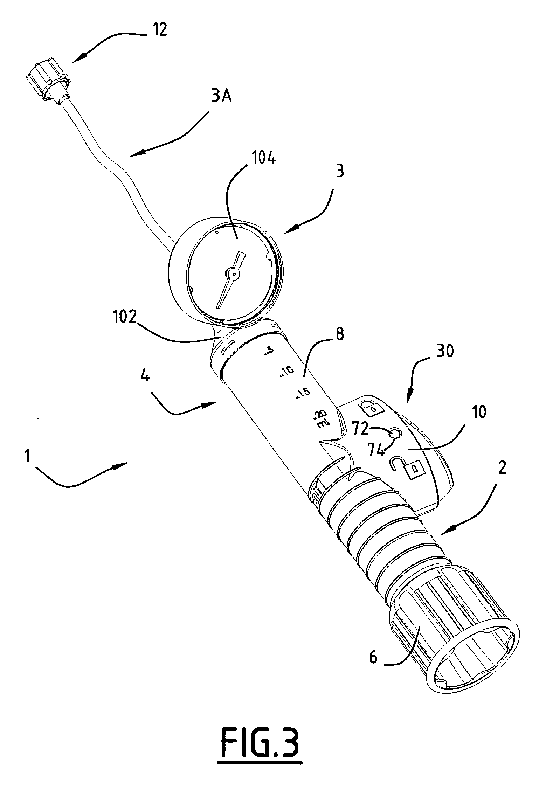

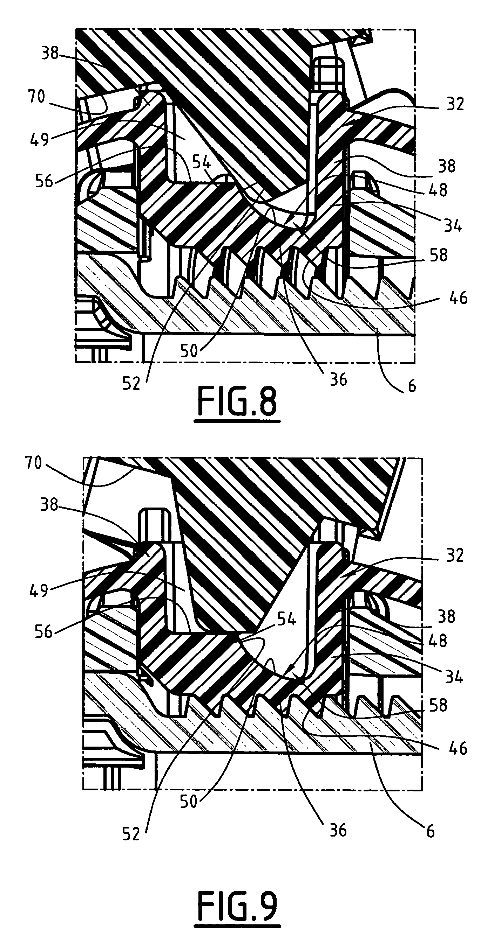

La demi-noix est ainsi déplaçable entre une position écartée du filetage 28, représentée sur les figures 2, 4, 5 et 8, dans laquelle le piston 6 est libre de coulisser dans le corps de seringue 4, et une position en prise avec ce filetage, représentée sur les figures 3 et 9, dans laquelle le piston 6 peut être vissé ou dévissé dans le corps de seringue 4, son libre coulissement étant impossible.

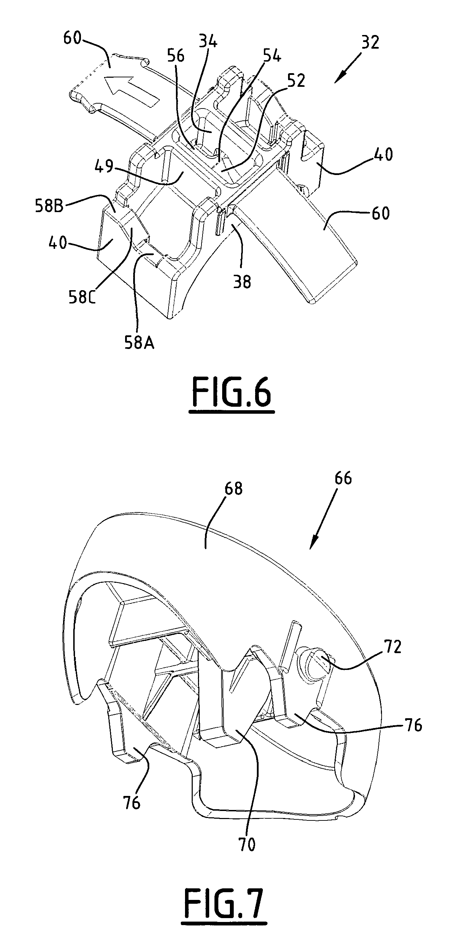

La paroi de fond 36 de la demi-noix 32 présente également, du côté dirigé vers l'extérieur du corps de seringue 4, une face étagée 48, représentée en détail sur les figures 8 et 9. Cette face est bordée par deux panneaux longitudinaux 49. Elle comporte successivement, d'arrière en avant, une première surface sensiblement plane 50 (non visible sur la figure 6), une surface inclinée vers l'extérieur formant rampe 52, un col 54 en saillie vers l'extérieur, et, en retrait vers l'intérieur par rapport au sommet du col 54, une deuxième surface sensiblement plane 56 plus éloignée de l'axe de seringue que la première surface 50. Sur les figures 8 et 9, les proportions respectives des surfaces 50, 52 et 56 et du col 54 sont exagérées pour plus de visibilité.

De même, les parois latérales opposées 40 présentent sur leur champ supérieur une succession de deux surfaces planes 58A, 58B ménagées à des niveaux différents reliées par une surface de came intermédiaire 58C. Ces surfaces sont agencées sensiblement suivant le même profil général que les surfaces 50, 56 et 52 respectivement.

L'ensemble de retenue 30 comporte également deux pattes déformables élastiquement 60 qui s'étendent de part et d'autre de la demi-noix 32. Ces pattes 60 sont venues de matière avec les parois latérales 38 du corps 34 de la demi-noix. Les extrémités libres de ces pattes sont élastiquement en appui le long du tronc 8 du corps de seringue.

Une cale 64 d'appui de la tige 20 est disposée en regard de la demi-noix 32 entre la paroi intérieure du corps de seringue et la tige 20 à l'opposé de la demi-noix 32 par rapport à l'axe de la tige.

L'ensemble de retenue 30 comporte en outre, comme représenté sur les figures 4, 5 et 7, un organe 66 de commande du déplacement de la demi-noix 32, entièrement réalisé en matière plastique, issue de moulage. Cet organe est formé d'une demi-coquille 68 dont l'évidement interne est tourné vers le corps de seringue 6, et d'un doigt 70 de forme générale pyramidale dont la base est venue de matière avec la demi-coquille.

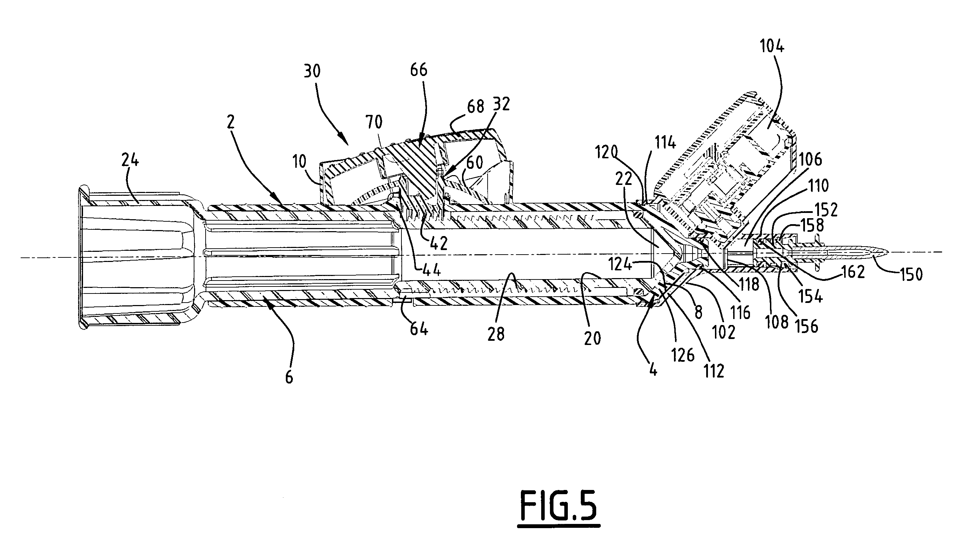

La demi-coquille 68 forme un capot de fermeture du boîtier 10 correspondant, en étant mobile par rapport à ce dernier. Plus précisément, comme représenté sur les figures 2 et 3, deux pions cylindriques 72 (un seul est visible sur les figures 2 et 3), d'axe perpendiculaire au plan de coupe des figures 4 et 5, sont formés en saillie par rapport à la demi coquille 68, chaque pion s'étendant depuis un des deux côtés de la demi-coquille sensiblement parallèles au plan de coupe de la figure 4. Ces pions sont reçus dans des ouvertures 74 sensiblement complémentaires ménagées dans la paroi du boîtier 10. De la sorte, l'organe de commande 66 est basculable autour de l'axe des pions par rapport au corps de seringue 4.

Le doigt 70 est adapté pour être reçu à l'intérieur du corps creux 34 de la demi-noix 32 et s'appuyer sur la face étagée 48 de cette demi-noix.

De manière correspondante, des doigts en saillie 76 sont formés de chaque coté de la demi-coquille 68 et sont propres coopérer avec les surfaces 58A, 58B, et 58C ménagées sur les parois latérales 40 de la demi-noix.

Plus précisément, lorsque l'organe de commande 66 est basculé vers l'arrière, comme représenté sur les figures 1, 4, 5 et 8, l'extrémité libre du doigt 70 est en appui contre la surface 50 et les doigts 76 sont en appui sur les surfaces 58A les pattes élastiques 60 maintenant la demi-noix en position écartée, de sorte que le piston 6 est libre de coulisser. Lorsque l'organe 66 est basculé vers l'avant, comme représenté sur les figures 3 et 9, le doigt 70 est en appui contre la surface 56, alors que les doigts 76 Sont en appui sur les surfaces 58B. Les pattes élastiques 60 sont sont alors déformées, de sorte que la demi-noix 32 est en prise avec le filetage 28 du piston pour n'autoriser que le vissage ou le dévissage de celui-ci.

Les déplacements du doigt 70 sont guidés, et éventuellement limités, par les faces internes des parois 38 et 49 du corps creux 34. A titre d'exemple, pour le dispositif représenté, l'angle de basculement du doigt est de l'ordre de 12° par rapport à l'axe de la seringue.

Le module de mesure de pression 3 comporte essentiellement une coiffe de support 102 rapportée sur le corps de seringue 4 et un manomètre 104 porté rigidement par la coiffe 102. Le manomètre est de tout type adapté et est par exemple un manomètre mécanique à aiguille, ou encore un manomètre électronique à sonde de pression.

La coiffe 102 délimite un conduit 106 visible sur les figures 4 et 5 dans lequel débouche radialement une prise de pression 108 en sortie de laquelle est connecté le manomètre 104.

La coiffe 102 présente une forme générale d'entonnoir présentant un nez généralement cylindrique 110 relié par un tronçon tronconique 112 à une jupe 114 entourant l'extrémité du tronc 8.

Le goulot 12 est muni, dans une gorge 116, d'un joint torique 118 assurant une étanchéité entre le corps de seringue et la coiffe 102.

En outre, à son extrémité, le tronc 8 du corps de seringue présente une gorge périphérique 120 propre à recevoir quatre saillies 122 ménagées sur la surface intérieure de la jupe 114. Les saillies 122 sont séparées angulairement de 90° à la périphérie de la jupe et assurent un guidage en rotation de la coiffe 102 autour de l'axe du tronc 8. Lors du montage, les saillies 122 sont engagées élastiquement autour du corps de seringue dans la gorge 120.

Le tronçon tronconique 118 de la coiffe comporte intérieurement des nervures axiales 124 espacées angulairement de 90°. Ces nervures sont visibles sur les figures 1 et 5. En outre, des butées d'arrêt 126 visibles sur la figure 4 sont prévues, extérieurement sur le tronçon convergent 14. Ces butées sont propres à coopérer avec les nervures 124 pour permettre un déplacement angulaire de la coiffe par rapport au corps de seringue sur une course maximale d'environ 90°.

La coiffe 120 est emmanchée par enclenchement élastique autour du corps de seringue 8, les saillies 122 étant reçues dans la gorge 120.

La tubulure 3A comporte un conduit souple 150 muni, à une première extrémité libre, d'un embout cylindrique 152 monté mobile à rotation dans un suralésage 154 du conduit 106 formé à l'extrémité libre du nez 110. Ce suralésage 154 présente une gorge 156 dans laquelle est reçue une collerette 158 de l'embout 152 pour permettre un maintien axial de l'embout dans le conduit. Un joint torique 162 ou tout autre système d'étanchéité est prévu dans l'embout 152 entre l'embout et la surface intérieur du conduit 106 pour permettre un guidage du fluide depuis le corps de seringue jusqu'au tuyau 150 au travers du conduit 106.

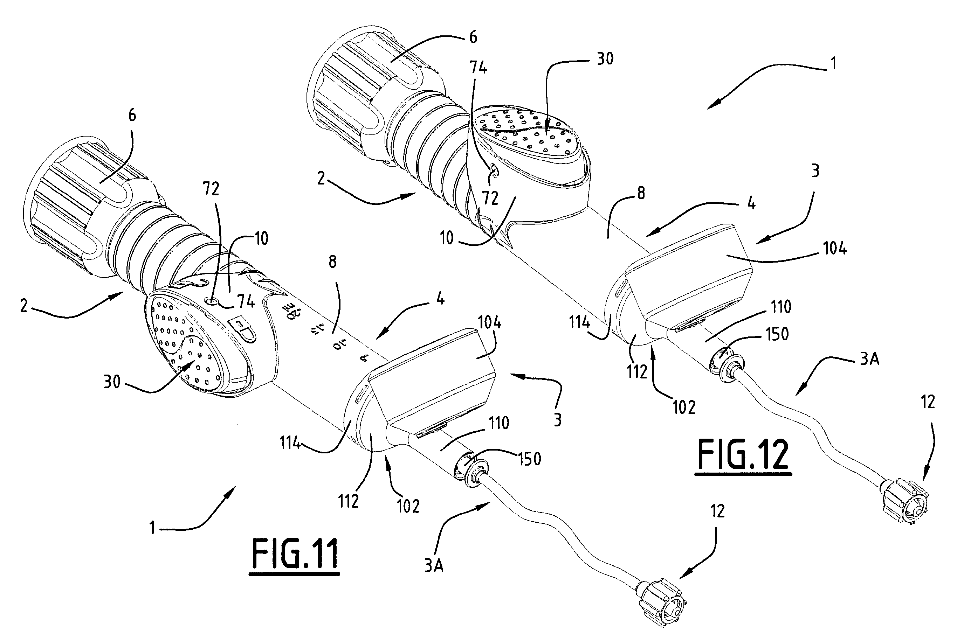

On conçoit qu'avec un tel dispositif, le manomètre 104 peut être placé par rapport au corps de seringue dans deux positions décalées angulairement de 90°, comme illustré aux figures 11 et 12, de sorte que le manomètre est, soit dans le même plan que celui de l'ensemble de retenue 30, comme illustré sur la figure 12, soit dans un plan décalé angulairement de 90° du plan de l'ensemble de retenue 30 autour de l'axe de la seringue, comme illustré sur la figure 11. Ainsi, quelque soit le mode de maniement de l'outil, que la poignée soit actionnée par la paume de la main ou par les doigts de la main, l'opérateur peut, en fonction de la position du manomètre, avoir un accès visuel aisé au manomètre pour lire la pression.

Enfin, la présence de la liaison rotative entre la tubulure 3A et le reste du dispositif permet un coût de fabrication réduit, la liaison rotative étant formée entre la coiffe 102 et le tuyau 150, ce qui permet de ne pas prévoir une liaison rotative à l'autre extrémité du tuyau 150. La position de la liaison rotative permet d'assembler la seringue et la tubulure à la fin de l'assemblage du dispositif.

Le fonctionnement du dispositif de gonflage 1 est le suivant :

En vue du gonflage d'un ballonnet, l'opérateur relie, par exemple par une tubulure pourvue à son extrémité amont d'un connecteur, l'extrémité avant du dispositif 1 à un ballonnet, par fixation du connecteur sur l'ensemble 12.

Il saisit ensuite le dispositif en pressant manuellement, notamment entre son pouce et son index, la partie arrière de la demi-coquille 68, de façon à faire basculer vers l'arrière l'organe de commande 66. L'opérateur peut alors pousser à l'intérieur du corps de seringue 4 la tige de piston 6 afin de faire augmenter la pression du fluide contenu dans la seringue, jusqu'à une pression de l'ordre de 3 bars, ce qui correspond généralement à la pression pouvant être obtenue par simple poussée du piston.

Après cela, l'opérateur bascule vers l'avant l'organe de commande 66 en exerçant une pression manuelle sur la partie avant de l'organe de commande 66. Le basculement vers l'avant de l'organe de commande provoque le déplacement de l'extrémité du doigt 70 depuis la surface 50 jusqu'à la surface 56. Cette extrémité s'appuie lors de son déplacement sur la rampe 52, en provoquant, par effet de came, la mise en prise du filet 28 par la demi-noix 32.

L'opérateur poursuit ensuite la montée en pression en vissant progressivement le piston dans le corps de seringue jusqu'à une pression pouvant atteindre, par exemple, 30 bars. La demi-noix 32 est fermement maintenue en prise avec le filetage du piston par le doigt 70, le col 54 de la face d'appui 48 de la demi-noix assurant le blocage des organes de commande en position basculée vers l'arrière.

La libération de la demi-noix s'effectue par la suite en répétant en sens inverse les étapes décrites ci-dessus.

Lors du fonctionnement du dispositif et notamment lors de la commande de l'organe de commande 30, le déplacement du doigt 70 s'accompagne du déplacement correspondant des doigts 76 le long des surfaces 58A, 58B et 58C. Ainsi, l'action du doigt 70 est renforcée par l'action simultanée de doigts 76. Les trois doigts étant répartis suivant la longueur de l'axe d'articulation de l'organe de commande 30, ils assurent une bonne stabilité de cette organe et une retenue efficace et fiable de la demi-noix en prise avec le filtage pour la retenue du piston, même si le piston exerce une force transversale importante sur la demi noix lors de son vissage.

Dans la seringue décrite ici, la présence de la cale 64 en regard de la demi-noix 32 disposée symétriquement évite tout fléchissement de la tige 20 du piston 6 et une retenue correcte de la tige par la demi-noix 32.

De plus, l'engagement et le désengagement de la demi-noix avec le filet 28 du piston 6 sont provoqués par le basculement de l'organe de commande 66 au moyen d'une prise en main ergonomique du dispositif.

## Revendications

1. Dispositif de gonflage de ballonnet, du type comportant une seringue (2) comprenant un corps de seringue (4) et un piston de seringue (6), formé d'une tige (20) munie, à une extrémité reçue à l'intérieur du corps de seringue (4), d'une tête (22) équipée d'un joint (23) qui coulisse de manière étanche à l'intérieur de ce corps de seringue (4), le piston de seringue (6) étant déplaçable à coulissement et à rotation dans ledit corps de seringue (4), le piston (6) présentant un filetage extérieur (28) sur au moins une partie de sa longueur, le corps de seringue comportant en outre un mécanisme escamotable (30) de retenue du piston (6) dans le corps de seringue, lequel mécanisme (30) est commutable entre un état escamoté dans lequel le piston (6) est libre de coulisser dans le corps de seringue et un état actif dans lequel le libre coulissement du piston (6) est impossible et dans lequel le piston (6) peut être vissé ou dévissé dans le corps de seringue (4),
le dispositif comportant un manomètre (104) de mesure de la pression en sortie du corps de seringue (4)
**caractérisé en ce que** le manomètre (104) est monté déplaçable angulairement par rapport au corps de seringue (4) et autour de l'axe du corps de seringue (4).

2. Dispositif selon la revendication 1, **caractérisé en ce qu'**il comporte une coiffe (102) délimitant un conduit (106) traversant dans lequel débouche une prise de pression (108), le manomètre (104) étant connecté en sortie de la prise de pression, et **en ce que** la coiffe (102) est montée déplaçable en rotation autour du corps de seringue (4).

3. Dispositif selon la revendication 2, **caractérisé en ce qu'**il comporte un joint torique d'étanchéité (23) disposé entre la coiffe (102) et le corps de seringue (4).

4. Dispositif selon la revendication 2 ou 3, **caractérisé en ce que** la coiffe (102) est engagée élastiquement autour du corps de seringue (4).

5. Dispositif selon l'une quelconque des revendications 2 à 4, **caractérisé en ce que** le corps de seringue (4) comporte un tronc (8) généralement cylindrique prolongé par un goulot (12) de diamètre extérieur inférieur à celui du tronc (8), et **en ce que** la coiffe (102) comporte une jupe (114) engagée au moins partiellement autour du tronc (8).

6. Dispositif selon l'une quelconque des revendications précédentes, **caractérisé en ce qu'**il comporte des butées (126) d'arrêt du manomètre (104) par rapport au corps de seringue (4) limitant le déplacement angulaire du manomètre à sensiblement 90°.

7. Dispositif selon l'une quelconque des revendications précédentes, **caractérisé en ce qu'**il comporte un module de pression (3) dans lequel le manomètre (104) est intégré, lequel module de pression (3) est monté déplaçable angulairement par rapport au corps de seringue (4), et autour du corps de seringue (4), et **en ce qu'**il comporte en outre une tubulure de sortie (3A) reliée au module de pression (3) par un embout rotatif (152) libre en rotation par rapport au module de pression (3).

8. Dispositif selon l'une quelconque des revendications précédentes, **caractérisé en ce que**
le corps de seringue comportant en outre un mécanisme escamotable (30) de retenue du piston (6) dans le corps de seringue, lequel mécanisme est commutable entre un état escamoté dans lequel le piston (6) est libre de coulisser dans le corps de seringue et un état actif dans lequel le libre coulissement du piston (6) est impossible et dans laquelle ce piston peut être vissé ou dévissé dans le corps de seringue (4),
**caractérisé en ce que** le mécanisme de retenue (30) comporte, pour la demi-noix (32), au moins un élément déformable élastiquement (60), en appui sur la demi-noix correspondante et sur le corps de seringue (4), et **en ce que** l'organe de commande (66) comporte au moins un doigt (70) d'appui sur la demi-noix correspondante (32), adapté pour s'appuyer, lors du déplacement de l'organe de commande, contre deux surfaces (50) portées par la demi-noix et décalées l'une par rapport à l'autre suivant une direction radiale au corps de seringue (4), la demi-noix étant dans sa position écartée du filetage lorsque le doigt est en appui contre la surface (50) la plus proche radialement du piston de seringue (6) et la demi-noix étant dans sa position en prise avec le filetage lorsque le doigt est en appui contre la surface (56) la plus éloignée.

## Patentansprüche

1. Vorrichtung zum Aufblasen eines Ballons von einer Art, welche eine Spritze (2) aufweist, welche einen Spritzenkörper (4) und einen Spritzenkolben (6) aufweist, welcher durch eine Stange (20) ausgebildet ist, welche an einem im Inneren des Spritzenkörpers (4) aufgenommenen Ende mit einem mit einer Dichtung (23) versehenen Kopf (22) ausgestattet ist, welcher sich auf dichte Art im Inneren des Spritzenkörpers (4) verschiebt, wobei der Spritzenkolben (6) in diesem Spritzenkörper durch Verschiebung und durch Drehung verlagerbar ist, wobei der Kolben (6) ein Außengewinde (28) mindestens auf einem Teil seiner Länge aufweist, wobei der Spritzenkörper ferner einen einklappbaren Kolben-Haltemechanismus (30) im Spritzenkörper aufweist, wobei dieser Mechanismus (30) umwandelbar ist zwischen einem eingeklappten Zustand, in welchem der Kolben (6) frei ist, sich im Spritzenkörper zu verschieben, und einem aktiven Zustand, in welchem das freie Verschieben des Kolbens (6) unmöglich ist, und in welchem der Kolben (6) in dem Spritzenkörper (4) hineingedreht oder herausgedreht werden kann,
wobei die Vorrichtung ein Manometer (104) zum Messen des Drucks am Ausgang des Spritzenkörpers (4) aufweist,
**dadurch gekennzeichnet, dass** das Manometer (104) winkelmäßig verlagerbar montiert ist in Bezug auf den Spritzenkörper (4), und um die Achse des Spritzenkörpers (4) herum montiert ist.

2. Vorrichtung gemäß Anspruch 1, **dadurch gekennzeichnet, dass** sie eine Abdeckung (102) aufweist, welche einen Durchgangskanal (106) begrenzt, in welchen eine Druckabnahme (108) mündet, wobei das Manometer (104) am Ausgang der Druckabnahme verbunden ist, und dass die Abdeckung (102) drehbar verlagerbar um den Spritzenkörper (4) herum montiert ist.

3. Vorrichtung gemäß Anspruch 2, **dadurch gekennzeichnet, dass** sie einen Dichtungs-O-Ring (23) aufweist, welcher zwischen der Abdeckung (102) und dem Spritzenkörper (4) angeordnet ist.

4. Vorrichtung gemäß Anspruch 2 oder 3, **dadurch gekennzeichnet, dass** die Abdeckung (102) elastisch um den Spritzenkörper (4) angebracht ist.

5. Vorrichtung gemäß einem der Ansprüche 2 bis 4, **dadurch gekennzeichnet, dass** der Spritzenkörper (4) einen im Allgemeinen zylindrischen Schaft (8) aufweist, welcher durch einen Engpass (12) fortgesetzt ist, dessen Außendurchmesser kleiner als der des Schafts (8) ist, und dass die Abdeckung (102) eine Schürze (114) aufweist, die mindestens teilweise um den Schaft (8) herumgreift.

6. Vorrichtung gemäß einem der vorhergehenden Ansprüche, **dadurch gekennzeichnet, dass** sie Anschläge (126) zum Arretieren des Manometers (104) in Bezug auf den Spritzenkörper (4) aufweist, welche die Winkeländerung des Manometers auf etwa 90° beschränken.

7. Vorrichtung gemäß einem der vorhergehenden Ansprüche, **dadurch gekennzeichnet, dass** sie ein Druckmodul (3) aufweist, in welchem das Manometer (104) integriert ist, wobei dieses Druckmodul (3) so montiert ist, dass sein Winkel in Bezug auf den Spritzenkörper änderbar ist und dass es um den Spritzenkörper (4) herum ist, und dass sie ferner eine Ausgangsleitung (3A) aufweist, welche mit dem Druckmodul mittels einer in einer Drehung in Bezug auf das Druckmodul (3) freien drehbaren Kappe (152) verbunden ist.

8. Vorrichtung gemäß einem der vorhergehenden Ansprüche, **dadurch gekennzeichnet, dass**
der Spritzenkörper ferner im Spritzenkörper einen einklappbaren Kolben-Haltemechanismus (30) aufweist, wobei dieser Mechanismus (30) umwandelbar ist zwischen einem eingeklappten Zustand, in welchem der Kolben (6) frei ist, sich im Spritzenkörper zu verschieben, und einem aktiven Zustand, in welchem das freie Verschieben des Kolbens (6) unmöglich ist, und in welchem der Kolben (6) in dem Spritzenkörper (4) hineingedreht oder herausgedreht werden kann,
**dadurch gekennzeichnet, dass** der Haltemechanismus (30) für die Halbnuss (32) mindestens ein elastisch verformbares Element (60) aufweist, welches sich auf die zugeordnete Halbnuss und auf den Spritzenkörper (4) stützt, und dass das Bedienteil (66) auf der zugeordneten Halbnuss (32) mindestens einen Stützfinger (70) aufweist, welcher dazu geeignet ist, sich bei der Verlagerung des Bedienteils gegen zwei Flächen (50) abzustützen, welche von der Halbnuss getragen werden, und welche eine in Bezug auf die andere in einer radialen Richtung des Spritzenkörpers (4) versetzt sind, wobei die Halbnuss in ihrer vom Gewinde entfernten Position ist, wenn der Finger sich gegen die Fläche (50) stützt, welche radial dem Spritzenkolben (6) am nächsten ist, und die Halbnuss in ihrer Eingriffsposition mit dem Gewinde ist, wenn der Finger sich gegen die am weitesten entfernte Fläche (56) stützt.

## Claims

1. Device for inflating a small balloon, of the type containing a syringe (2) comprising a syringe body (4) and a syringe piston (6), formed of a rod (20) fitted, at one end received inside the syringe body (4), with a head (22) equipped with a joint (23) which slides tightly inside this syringe body (4), the syringe piston (6) being movable on sliding and on rotation in said syringe body (4), the piston (6) having an external threading (28) on at least part of its length, the syringe body furthermore containing a retractable mechanism (30) for retaining the piston (6) in the syringe body, which mechanism (30) can be switched between a retracted state in which the piston (6) is free to slide in the syringe body and an active state in which the free sliding of the piston (6) is impossible and in which the piston (6) can be screwed or unscrewed in the syringe body (4), the device containing a pressure gauge (104) for measuring the pressure at the outlet of the syringe body (4), **characterised in that** the pressure gauge (104) is mounted to be able to move at an angle in relation to the syringe body (4) and around the axis of the syringe body (4).

2. Device according to claim 1, **characterised in that** it contains a cap (102) delimiting a channel (106) passing through into which a pressure tap (108) opens, the pressure gauge (104) being connected at the outlet of the pressure tap, and **in that** the cap (102) is mounted to be able to move in rotation around the syringe body (4).

3. Device according to claim 2, **characterised in that** it contains a toric sealing joint (23) arranged between the cap (102) and the syringe body (4).

4. Device according to claim 2 or 3, **characterised in that** the cap (102) is elastically fitted around the syringe body (4).

5. Device according to any one of claims 2 to 4, **characterised in that** the syringe body (4) contains a generally cylindrical shank (8) extended by a neck (12) of external diameter less than that of the shank (8), and **in that** the cap (102) contains a skirt (114) fitted at least partially around the shank (8).

6. Device according to any one of the preceding claims, **characterised in that** it contains end stops (126) on the pressure gauge (104) in relation to the syringe body (4) limiting the angular movement of the pressure gauge to substantially 90°C.

7. Device according to any one of the preceding claims, **characterised in that** it contains a pressure module (3) in which the pressure gauge (104) is integrated, which pressure module (3) is mounted to be able to move at an angle in relation to the syringe body (4) and around the syringe body (4), and **in that** it furthermore contains an outlet pipe (3A) linked to the pressure module (3) by a rotary nozzle (152) freely rotating in relation to the pressure module (3).

8. Device according to any one of the preceding claims, **characterised in that**
the syringe body furthermore containing a retractable mechanism (30) for retaining the piston (6) in the syringe body, which mechanism can be switched between a retracted state in which the piston (6) is free to slide in the syringe body and an active state in which the free sliding of the piston (6) is impossible, and in which this piston can be screwed or unscrewed in the syringe body (4),
**characterised in that** the retaining mechanism (30) contains, for the half-nut (32), at least an elastically deformable element (60) pressing on the corresponding half-nut and on the syringe body (4), and **in that** the control element (66) contains at least a support finger (70) on the corresponding half-nut (32), adapted to press, during the movement of the control element, against two surfaces (50) provided by the half-nut and moved one in relation to the other following a radial direction to the syringe body (4), the half-nut being in its deflected threading position when the finger presses against the radially closest surface (50) of the syringe piston (6) and the half-nut being in its engage position with the threading when the finger presses against the furthest surface (56).
